# EUROPÄISCHE PATENTANMELDUNG

(11) **EP 2 138 130 A1**
(43) Veröffentlichungstag der Anmeldung: **30.12.2009**
(21) Anmeldenummer: 09170163.1
(22) Anmeldetag: 14.06.2004
(51) Int. Cl.: A61F 2/06, A61L 31/16, A61L 31/10, A61F 2/82

(54) **Beschichteter Stent mit axial variierender Elutionscharakteristik**

(30) Priorität: 23.06.2003 DE 10329260
(62) Teilanmeldung aus: 04739861.5
(71) Anmelder: BIOTRONIK GmbH & Co. KG, 12359 Berlin (DE)
(72) Erfinder: Harder, Claus, 91080, Uttenreuth (DE); Delaloye, Stephane, 8180, Bülach (CH); Heublein, Bernd, verstorben (DE)
(74) Vertreter: Lindner-Vogt, Karin L.

(57) **Zusammenfassung**

Ein Stent (10) mit einem rohrförmigen, an seinen Stirnseiten offenen Grundkörper (14), dessen Umlaufswandung (16) zumindest bereichsweise mit einem Beschichtungssystem (26) aus einem oder mehreren polymeren Trägern und wenigstens zwei pharmakologisch wirksamen Substanzen bedeckt ist, wobei die Substanzen nach Implantation des Stents (10) in den menschlichen oder tierischen Körper in das umgebende Gewebe freigesetzt wird, ist **dadurch gekennzeichnet, dass** die Konzentration der wenigstens zwei pharmakologischen Substanzen in Längsrichtung des Stents (10) derart vorgegeben ist, dass die Substanz eine in Abhängigkeit von den in der Applikation zu erwartenden pathophysiolgischen und/oder rheologischen Verhältnissen vorgegebene, in Längsrichtung des Stents (10) lokal unterschiedliche Elutionscharakteristik aufweist.

## Beschreibung

Die Erfindung betrifft Stents mit Beschichtungssystemen aus einem oder mehreren polymeren Trägern und wenigstens einer pharmakologisch wirksamen Substanz, wobei die Substanz nach Implantation des Stents in den menschlichen oder tierischen Körper in das umgebende Gewebe freigesetzt wird.

Koronare Herzerkrankungen, insbesondere akute Myokardinfarkte, stellen in Westeuropa und Nordamerika eine der häufigsten Todesursachen dar. In mehr als 80% der Fälle ist die Ursache des Myokardinfarktes der thrombotische Verschluss einer Koronararterie durch Ruptur einer atheromatösen Plaque bei vorbestehender stenosierender Atheromatose.

Entscheidende Faktoren für die Langzeitprognose nach akutem Myokardinfarkt sind:
- eine effektive und langanhaltende Wiedereröffnung der Infarktarterie,
- eine Dauer des thrombotischen Gefäßverschlusses,
- die Verhinderung eines größeren Myokardverlustes und eines ventrikulären Remodeling,
- die Beherrschung rhythmogener Komplikationen.

Die genannten Faktoren bestimmen nicht nur die kardiovaskuläre Mortalität, sondern auch die Lebensqualität nach dem Infarkt.

Seit mehr als zwanzig Jahren sind nicht-operative Methoden zur Stenose-Behandlung etabliert, bei denen u. a. durch Ballondilatation (PTCA Perkutane Transluminale Coronare Angioplastie) das verengte oder verschlossene Blutgefäß wieder aufgeweitet wird. Dieses Vorgehen hat sich insbesondere bei der Therapie des akuten Myokardinfarktes bewährt. Mit dem Aufweiten des Blutgefäßes entstehen allerdings kleinste Verletzungen, Einrisse, Dissektionen in der Gefäßwand, die zwar häufig problemlos verheilen, jedoch in etwa einem Drittel der Fälle durch das ausgelöste Zellwachstum zu Wucherungen führen (Proliferation), die letztendlich zu einer erneuten Gefäßverengung (Restenose) führen. Die Aufweitung beseitigt auch nicht die Ursachen der Stenose, also die physiologischen Veränderungen in der Gefäßwand. Eine weitere Ursache der Restenose ist die Elastizität des gedehnten Blutgefäßes. Nach dem Entfernen des Ballons zieht sich das Blutgefäß übermäßig zusammen, so dass der Gefäßquerschnitt verringert wird (Obstruktion, sogenanntes negatives remodeling). Letzterer Effekt kann nur durch Platzierung eines Stents vermieden werden.

In der interventionellen Therapie der stabilen Angina pectoris bei koronarer Herzkrankheit, hat die Einführung der Stents zu einer deutlichen Reduktion der Rate an Restenosen und damit zu besseren Langzeitresultaten geführt. Dies gilt sowohl für die primäre als auch die Rezidivstenose. Ursächlich für den Nutzen der Stent-Implantation ist der höhere primäre Lumengewinn.

Durch den Einsatz von Stents kann zwar ein optimaler Gefäßquerschnitt erreicht werden, allerdings führt der Einsatz von Stents ebenfalls zu kleinsten Verletzungen, die die Proliferation induzieren können und damit letztendlich eine Restenose auslösen können. Weiterhin initiiert die Anwesenheit eines derartigen Fremdkörpers eine Kaskade von mikrobiologischen Prozessen, die zu einem allmählichen Zuwachsen des Stents führen können.

Mittlerweile bestehen umfangreiche Erkenntnisse zum zellbiologischen Mechanismus und zu den auslösenden Faktoren der Stenose und Restenose. Die Restenose entsteht - wie bereits erläutert - als Reaktion der Gefäßwand auf die lokale Verletzung infolge Dehnung des atherosklerotischen Plaque. Über komplexe Wirkmechanismen wird die lumengerichtete Migration und Proliferation der glatten Muskelzellen der Media und der Adventitia induziert (neointimale Hyperplasie). Unter Einfluss verschiedener Wachstumsfaktoren produzieren die glatten Muskelzellen eine Deckschicht aus Matrixproteinen (Elastin, Kollagen, Proteoglykane), deren ungesteuertes Wachstum allmählich zu einer Einengung des Lumens führen kann. Systemische medikamentöse Therapieeinsätze sehen u.a. die orale Verabreichung von Calzium-Antagonisten, ACE-Hemmern, Antikoagulantien, Antiaggregantien, Fischölen, antiproliferativen Substanzen, antiinflammatorischen Substanzen und Seroto-nin-Antagonisten vor, signifikante Reduktionen der Restenosearten wurden auf diesem Wege bisher jedoch nicht erreicht.

Seit einigen Jahren versucht man die Restenosegefahr bei der Implantation von Stents durch Aufbringung spezieller Beschichtungssysteme zu mindern. Teilweise dienen die Beschichtungssysteme als Träger, in die ein oder mehrere pharmakologisch wirksame Subtanzen eingebettet sind (Local Drug Delivery, LDD). Die Beschichtungssysteme bedecken in der Regel zumindest eine der Gefäßwand zugewandte Umlaufswandung des endovaskulären Implantates. Als Wirkstoffe oder Wirkstoffkombinationen für LDD-Systeme wurden bisher zahlreiche Präparate vorgeschlagen.

Der Träger derartiger Beschichtungssysteme besteht aus einem biokompatiblen Material, welches entweder natürlichen Ursprungs ist oder auf synthetischem Wege gewonnen werden kann. Eine besonders gute Verträglichkeit und die Möglichkeit, die Elutionscharakteristik des eingebetteten Arzneistoffs zu beeinflussen bieten biodegradierbare Beschichtungsmaterialien. Beispiele für die Verwendung biodegradierbarer Polymere sind Cellulose, Kollagen, Albumin, Casein, Polysaccharide (PSAC), Polylactid (PLA), Poly-L-lactid (PLLA), Polyglykol (PGA), Poly-D,L-lactid-co-glycolid (PDLLA/PGA), Polyhydroxybuttersäure (PHB), Polyhydroxyvaleriansäure (PHV), Polyalkylcarbonate, Polyorthoester, Polyethylenterephthalat (PET), Polymalonsäure (PML), Polyanhydride, Polyphosphazene, Polyaminosäuren und deren Copolymere sowie Hyaluronsäure und seine Derivate.

Zur Aufbringung der Beschichtungssysteme auf den Stent sind zahlreiche Verfahren entwickelt worden, wie beispielsweise Rotationszerstäubungsverfahren, Tauchverfahren und Sprühverfahren. Das Beschichtungssystem bedeckt zumindest bereichsweise die der Gefäßwand zugewandte Umlaufswandung des Stents. Eine Freisetzung der pharmakologisch wirksamen Substanzen erfolgt im menschlichen bzw. tierischen Körper durch allmähliche Degradation des Trägers und/oder Diffusion in das umgebende Gewebe. Die Elutionscharakteristik der Substanzen lässt sich mit Hilfe etablierter in vitro Untersuchungen vorab abschätzen.

Bekannte LDD-Stents zeigen keine lokal differenzierte Elutionscharakteristik für die Substanzen. So sind die Beschichtungssysteme im Bereich der offenen Stirnseiten des rohrförmigen Grundkörpers des Stents als auch in mittleren Bereichen des Stents von annähernd gleicher Beschaffenheit. Gerade bei langgestreckten Stenosen, bei denen sich der Läsionscharakter über die Stentlänge ändert, können derartige Beschichtungssysteme allerdings nachteilig sein. Dies kann beispielsweise der Fall sein bei einer langgestreckten, mit einer bestimmten Substanz zu behandelnden Läsion, deren Plaquemenge im Zentrum sehr hoch ist und nach außen hin abnimmt. Bei homogener substantieller Therapie werden unter Umständen die Randbereiche des Stents überdosiert, was in diesen Bereichen zu einem Proliferationsreiz führen kann, während dieselbe Dosierung im Mittelbereich der Läsion antiproliferativ wirkt. Weiterhin ist ein Austrag der Substanzen eines LDD-Stents an seinen Enden, d. h. im Bereich seiner offenen Stirnseiten, erhöht, was zu einer lokalen Unterdosierung führen kann.

Ausgehend vom Stand der Technik stellt sich damit die Aufgabe, ein Beschichtungssystem zu schaffen, mit dem eine optimale lokale Wirkstoffapplikation über die gesamte Länge des Stents möglich wird.

Diese Aufgabe wird durch den Stent mit den in Anspruch 1 genannten Merkmalen gelöst.

Die Erfindung geht aus von einem Stent mit einem rohförmigen, an seinen Stirnseiten offenen Grundkörper, dessen Umlaufswandung zumindest bereichsweise mit einem Beschichtungssystem aus einem oder mehreren polymeren Träger und wenigstens einer pharmakologisch wirksamen Substanz bedeckt ist. Der erfindungsgemäße Stent zeichnet sich dadurch aus, dass ein oder mehrere Parameter des Beschichtungssystems, nämlich
- eine Konzentration der Substanz,
- eine morphologische Struktur des oder der Träger,
- eine stoffliche Modifikation des oder der Träger und/oder
- eine Schichtdicke des oder der Träger,
in Längsrichtung des Stents derart vorgegeben sind, dass die Substanz eine in Abhängigkeit von den in der Applikation zu erwartenden pathophysiologischen und/oder rheologischen Verhältnissen vorgegebene, in Längsrichtung des Stents lokal unterschiedliche Elutionscharakteristik aufweist. Hierdurch ist es möglich, die wenigstens eine Substanz über die Länge des Stents in unterschiedlichem Maße in das anliegende Gewebe freizusetzen.

Unter "Beschichtungssystem" in Sinne der Erfindung wird die Kombination aus einem polymeren, gegebenenfalls biodegradierbaren Träger mit zumindest einer pharmakologisch wirksamen Substanz verstanden. Das Beschichtungssystem bedeckt zumindest bereichsweise eine äußere Oberfläche des Stents.

Unter "pharmakologisch wirksame Substanz" wird ein Arzneimittel verstanden, dass in geeigneter Dosierung als Therapeutikum zur Beeinflussung von Zuständen oder Funktionen des Körpers, als Ersatz für natürliche von menschlichen oder tierischen Körper erzeugte Wirkstoffe sowie zu Beseitigung oder Unschädlichmachung von Krankheitserregern oder körperfremden Stoffen dient.

Unter "lokaler Elutionscharakteristik" wird die Freisetzung einer Substanz in die benachbarte Gewebsumgebung über einen bestimmten Zeitraumraum verstanden und zwar räumlich begrenzt auf einen vorgegebenen Teilbereich des beschichteten Stents.

Ist beispielsweise die Konzentration der Substanz in einem mittleren Abschnitt des Stents erhöht, so ist die lokale Dosierung in diesem Abschnitt ebenfalls erhöht. Erstreckt sich in diesem Abschnitt des Stents eine lokale Läsion, so kann sie hochpotent mit optimaler Dosierung behandelt werden. In Richtung der Stirnseite nimmt die Dosierung der Substanz dagegen ab, so dass Proliferationsreize vermieden werden.

Andererseits ist tendenziell eine Neointimabildung an den Enden des Stents erhöht. Es ist daher sinnvoll, in diesen Bereichen Beschichtungssysteme zu etablieren, die eine die Neointimabildung hemmende oder unterdrückende Substanz in gegenüber den mittleren Bereichen des Stents erhöhter Konzentration aufweisen. Dadurch wird die Dosis der Substanz in dem an den Enden gegenüberliegenden Gefäßgewebe erhöht.

Durch Variation der Schichtdicke des Trägers kann ebenfalls die lokale Elutionscharakteristik über die Länge des Stents beeinflusst werden. Hier steht im Vordergrund, die Dosierung über einen bestimmten Zeitraum aufrecht zu erhalten. So ist es je nach pathophysiologischen Verhältnissen in den einzelnen, der Umlaufswandung des Stents gegenüberliegenden Gefäßabschnitten notwendig, die medikamentöse Behandlung über einen bestimmten Zeitraum aufrecht zu erhalten. Mit einer erhöhten Schichtdicke kann der Dosierungszeitraum verlängert werden. Selbstverständlich können je nach Applikation Schichtdicke, morphologische Struktur, stoffliche Modifizierung als auch Konzentration der Substanz variiert werden.

Unter "morphologischen Strukturen" im erfindungsgemäßen Sinne wird die Konformation und Aggregation der den Träger bildenden Polymere verstanden. Dies beinhaltet den Typ der molekularen Ordnungsstruktur, die Porosität, die Oberflächenbeschaffenheit und andere intrinsische Eigenschaften des Trägers, die eine Diffusion des Wirkstoffs oder das Degradationsverhalten des Trägers beeinflussen. Molekulare Ordnungsstrukturen umfassen amorphe, (teil-)kristalline oder mesomorphe Polymerphasen, die in Abhängigkeit von dem jeweils eingesetzten Herstellungsverfahren, Beschichtungsverfahren und Umweltbedingungen beeinflussbar bzw. erzeugbar sind. Durch gezielte Variation des Herstellungs- und Beschichtungsverfahrens kann die Porosität und die Oberflächenbeschaffenheit des Trägers beeinflusst werden. Generell gilt, dass mit zunehmender Porosität des Trägers der Wirkstoff schneller freigesetzt wird. Amorphe Strukturen zeigen gegenüber (teil-)kristallinen Strukturen ähnliche Effekte.

Unter "stofflicher Modifizierung" wird hier die Blendherstellung aus Polymeren sowie die Zugabe von Füll- und Zusatzstoffen (Additiven) zum Zwecke der Beeinflussung der Elutionscharakteristik verstanden werden.

Vorzugsweise ist der Träger aus einem biodegradierbaren Polymer geformt, so dass die Substanz nach Implantation des Stents in den menschlichen oder tierischen Körper auch durch allmähliche Degradation des Trägers in das umgebende Gewebe freigesetzt wird. Das Degradationsverhalten des Trägers stellt damit eine weitere Größe dar, mit der die Wirkstofffreisetzung kontrolliert werden kann, d. h. mit der eine Differenzierung der Elutionscharakteristik im erfindungsgemäßen Sinne möglich ist. Ein schneller Abbau des Trägers führt zu einer schnellen Freisetzung der Substanz. Die Abbaugeschwindigkeit des biodegradierbaren Polymers ist nicht nur von dem jeweils vorliegenden polymeren Trägermaterial abhängig, sondern kann auch durch Variation der morphologischen Struktur und durch stoffliche Modifizierung beeinflusst werden.

Die lokale Elutionscharakteristik der Substanz wird erfindungsgemäß in axialer Richtung, d. h. über die Länge des Stents, in Abhängigkeit von den in der Applikation zu erwartenden pathophysiologischen und/oder rheologischen Verhältnissen vorgegeben. Die pathophysiologischen Aspekte tragen dem Umstand Rechnung, dass in der Regel der Stent derart im Gefäß plaziert wird, dass er mittig an der Läsion anliegt, d. h. das anliegende Gewebe an den Enden und im mittleren Bereich des Stents unterschiedlicher Beschaffenheit ist. Rheologische Aspekte tragen wiederum dem Umstand Rechnung, dass die Strömungsverhältnisse, insbesondere im Bereich der Enden und in mittleren Abschnitten des Stents unterschiedlich sind. So kann es an den Enden des Stents zu einer vermehrten Austragung der Substanz aufgrund stärkerer Strömung kommen. Rheologische Parameter können insbesondere durch Vorgabe des Stentdesigns stark variieren und müssen im Einzelfall bestimmt werden. Durch Berücksichtigung der beiden genannten Parameter kann eine für die LDD-Therapie optimale Dosierung über die gesamte Dimension des Stents sichergestellt werden.

Vorzugsweise wird zusätzlich das Freisetzungsverhalten verschiedener polymerer Träger mit einbezogen. Für den Fall, dass ein oder mehrere der Träger biodegradierbar sind, kann zur Variation der lokalen Elutionscharakteristik auch das Degradationsverhalten des oder der Träger in der schon zuvor geschilderten Art und Weise beeinflusst werden. So kann beispielsweise zur Erhöhung der lokalen Dosierung ein im Vergleich zu den anderen vorhandenen Trägern schneller abbaubarer Träger in einem bestimmten Bereich des Stents vorgesehen sein. Der schnellere Abbau des Trägers in diesem Bereich führt zu einer lokalen Erhöhung der Dosierung der an sich in gleicher Konzentration auch in den anderen Trägern vorhandenen Substanz. Ein solches System bietet sich beispielsweise dann an, wenn eine Erhöhung der Konzentration der Substanz im Trägermaterial zu unerwünschten Kristallisationsprozessen führt, die wiederum das Freisetzungsverhalten und die Langzeitstabilität negativ beeinflussen.

Die Darstellung des erfindungsgemäßen Beschichtungssystems kann unter Rückgriff auf konventionelle Beschichtungstechniken erfolgen. Zur lokalen Aufbringung können gängige Maskierungstechniken genutzt werden.

Die Erfindung wird nachfolgend anhand von Ausführungsbeispielen und den dazugehörigen Zeichnung näher erläutert. Es zeigen:
- Fig. 1: einen Stent mit einem rohrförmigen, an seinen Stirnseiten offenen Grundkörper, dessen Umlaufswandung mit einem Beschichtungssystem bedeckt ist;
- Fig. 2a, 2b: einen schematischen Querschnitt entlang einer Längsachse eines Stents zur Illustration des erfindungsgemäßen Beschichtungssystems nach einer ersten Variante;
- Fig. 3a, 3b: einen schematischen Querschnitt entlang einer Längsachse eines Stents zur Illustration des erfindungsgemäßen Beschichtungssystems nach einer zweiten Variante;
- Fig. 4: einen schematischen Querschnitt entlang einer Längsachse eines Stents zur Illustration des erfindungsgemäßen Beschichtungssystems nach einer dritten Variante;
- Fig. 5: einen schematischen Querschnitt entlang einer Längsachse eines Stents zur Illustration des erfindungsgemäßen Beschichtungssystems nach einer vierten Variante.

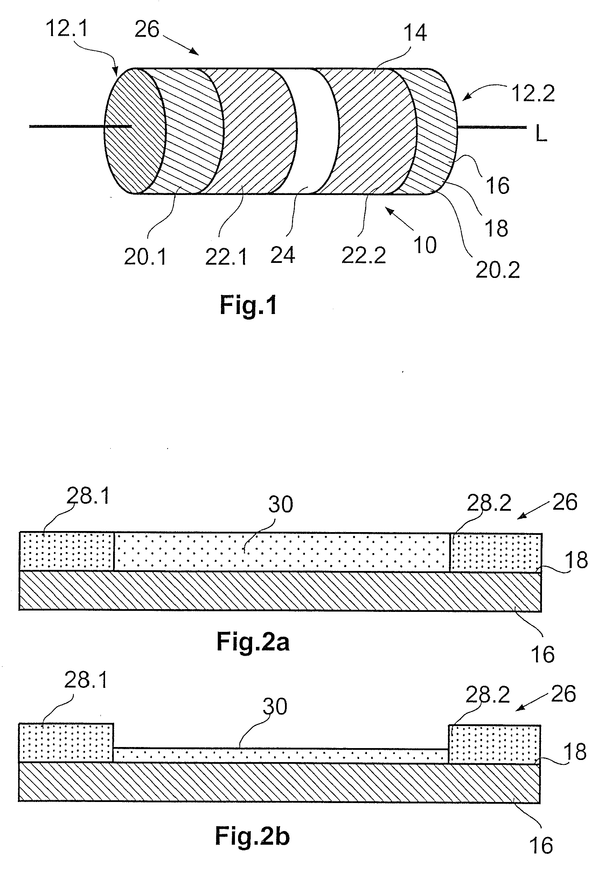

Die Fig. 1 zeigt stark schematisiert eine perspektivische Seitenansicht auf einen Stent 10 mit einem rohrförmigen, an seinen Enden 12.1, 12.2 offenen Grundkörper 14. Eine sich radial um eine Längsachse L erstreckende Umlaufswandung 16 des Grundkörpers 14 besteht aus in axialer Richtung nebeneinander angeordneten Segmenten, die sich wiederum aus einer Vielzahl von in einem bestimmten Muster angeordneten Stützelementen zusammensetzen. Die einzelnen Segmente werden über Verbindungsstege miteinander verbunden und ergeben zusammengefasst den Grundkörper 14. In der Fig. 1 wurde bewusst auf die Darstellung eines bestimmten Stentdesigns verzichtet, da dies zu Zwecken der Darstellung des erfindungsgemäßen Beschichtungssystems nicht notwendig ist und zudem für jedes Stentdesign eine individuelle Anpassung des Beschichtungssystems an die jeweils gegebenen geometrischen Faktoren und anderen Parameter notwendig ist. Stentdesigns unterschiedlichster Ausbildung sind in sehr großer Vielfalt aus dem Stand der Technik bekannt und werden daher hier nicht näher erläutert. Festzuhalten bleibt lediglich, dass alle gängigen Stents 10 ein wie auch immer geartetes rohrförmiges Grundgerüst 14 aufweisen, das eine umlaufende Umlaufswandung 16 umfasst. Im Folgenden wird daher eine äußere Mantelfläche 18 der Umlaufswandung 16 mit der sich ggf. aus einer Vielzahl von vorhandenen Stützelementen gebildeten äußeren Umlauffläche dieser Stützelemente gleichgesetzt.

Der Stent 10 der Fig. 1 zeigt in stark schematisierter Weise ein Beschichtungssystem 26, bei dem mehrere Abschnitte 20.1, 20.2, 22.1, 22.2, 24 der äußeren Mantelfläche 18 der Umlaufswandung 16 mit in ihren Eigenschaften divergierenden Beschichtungen versehen sind.

Die Unterschiede der Beschichtungen in den einzelnen Abschnitten 20.1, 20.1, 20.2, 22.1, 22.2, 24 bestehen darin, dass sich die aus biodegradierbaren Träger und pharmakologisch wirksamer Substanz bestehenden einzelnen abschnittsweise Beschichtungen in ihrer lokalen Elutionscharakteristik für die pharmakologisch wirksame Substanz unterscheiden. So kann - wie im einzelnen noch näher erläutert wird - vorgesehen sein, dass die Abschnitte 20.1 und 20.2 an den Enden 12.1, 12.2 des Stents 10 die Substanz über die Zeit mit einer ersten Dosierung freisetzen, die für diese Substanz höher liegt als in den stärker in der Mitte angeordneten Abschnitten 22.1, 22.2 und 24. Dies hat wiederum zur Folge, dass die nach einer Implantation den jeweiligen Abschnitten 20.1, 20.2, 22.1, 22.2 und 24 gegenüberliegenden Gewebsbereiche der Gefäßwand einer unterschiedlichen Dosierung der Substanz ausgesetzt sind. In jedem Fall weist das Beschichtungssystem also zwei oder mehr Abschnitte mit einer lokal unterschiedlichen Elutionscharakteristik für die Substanz auf.

Die Fig. 2a, 2b, 3a, 3b, 4 und 5 zeigen - in jeweils stark schematisierter Weise - einen Schnitt entlang der Längsachse L des Stents 10 und zwar jeweils nur einen der sich dabei ergebenen zwei Schnitte durch die Umlaufswandung 16. Zuvor wird jedoch kurz auf die zugrunde liegenden Prinzipien bei der Ausgestaltung der einzelnen Beschichtungssysteme eingegangen.

Eine lokale Elutionscharakteristik einer oder mehrerer in dem Beschichtungssystem vorhandener Substanzen hängt im Wesentlichen von fünf Faktoren ab:
a) einer Konzentration der Substanzen in dem oder mehreren Trägern,
b) einer Schichtdicke der Träger,
c) einem Degradationsverhalten der Träger,
d) einer morphologischen Struktur der Träger und
e) einer stofflichen Modifizierung der Träger.

Mit Punkt a) wird dem grundsätzlichen Prinzip Rechnung getragen, dass mit Erhöhung der Wirkstoffkonzentration auch eine höhere Dosierung einhergeht. Dieses Phänomen muss allerdings nicht unbedingt linear verlaufen und sowohl Dosis als auch Freisetzungsdauer werden von weiteren Faktoren mit beeinflusst. Das Prinzip der Wirkstofffreisetzung durch Diffusion ist aber sowohl theoretisch als auch praktisch an zahlreichen Beispielen untermauert worden, so dass zum einen theoretische Voraussagen zur in vivo Freisetzung möglich sind und zum anderen in vitro Experimente mit hoher Treffgenauigkeit die im Körper tatsächlich stattfindenden Prozesse simulieren können.

Eine Variation der Schichtdicke des Trägers (Punkt b)) bei gleich bleibender Konzentration der eingebetteten Substanz beeinflusst in der Regel die Dosierungsdauer. Es können allerdings auch weitere Effekte an den Phasengrenzflächen auftreten, die sich zusätzlich auf die Freisetzung der Substanz und damit die Dosis der Substanz in einem bestimmten Zeitintervall auswirken. Auch hierzu bestehen fundierte theoretische als auch praktische Modellsysteme, die eine Abschätzung des späteren in vivo Verhaltens ermöglichen.

Ein weiterer, die lokale Elutionscharakteristik beeinflussender Faktor, ist das Degradationsverhalten des biodegradierbaren Trägers (Punkt c)). Mit dem allmählichen Abbau des Trägers wird die in diesen Bereichen eingebettete Substanz freigesetzt. In der Regel laufen hierzu parallel Diffusionsprozesse. Je nach Löslichkeit der Substanz kann es aber durchaus sein, dass die Degradation des Trägers sehr viel rascher fortschreitet als die allmähliche Auflösung der Substanz. So kann die Substanz unter Umständen noch als Mikropartikel oder Nanopartikel von dem umgebenden Gewebe aufgenommen werden. Über das Degradationsverhalten einzelner Trägersysteme liegen bereits fundierte wissenschaftliche Erkenntnisse vor. Ausgehend hiervon und von dazu parallelen in vitro Versuchen kann das Verhalten gleichartiger Trägersysteme im lebenden Organismus vorausgesagt werden.

Schließlich hängt die lokale Elutionscharakteristik von der morphologischen Struktur und stofflichen Modifikationen der Träger ab (Punkte d) und e)). So kann insbesondere die Porosität der Träger unterschiedlich sein, wobei eine höhere Porosität zu einer beschleunigten Degradation und einer erhöhten Diffusion führt. Zur stofflichen Modifikation kann beispielsweise vorgesehen sein, Additive den Trägern beizumengen, die den enzymatischen Abbau verzögern.

Zusammenfassend ist daher festzuhalten, dass je nach Variabilität des Systems, d. h. ob beispielsweise mehrere Trägersysteme vorhanden sind, oder die Konzentrationen der ein oder mehreren Substanzen sich in einzelnen Abschnitten des Stents ändert oder die Schichtdicken der Träger geändert werden, eine lokale Elutionscharakteristik der einen oder mehreren Substanzen einstellbar ist.

Eine Anpassung der einzelnen Abschnitte des Beschichtungssystems des Stents wird dabei in Abhängigkeit von den in der Applikation zu erwartenden pathophysiologischen und rheologischen Verhältnissen durchgeführt.

Unter den pathophysiologischen Verhältnissen wird hier die durch Krankheit in dem gestenteten Gefäßbereich veränderte Gewebsstruktur verstanden. In der Regel wird der Stent so platziert, dass die Läsion, d. h. bei koronaren Applikationen in der Regel die fibroatheromatöse Plaque, etwa im mittleren Bereich des Stents liegt. Mit anderen Worten, die anliegenden Gewebsstrukturen divergieren in axialer Richtung über die Länge des Stents und damit ist auch unter Umständen lokal eine andere Behandlung indiziert.

Unter den rheologischen Verhältnissen werden die Strömungsverhältnisse verstanden, wie sie sich nach Implantation des Stents in den einzelnen Längsabschnitten des Stents einstellen. Erfahrungsgemäß hat sich gezeigt, dass die Enden des Stents stärker als die mittleren Bereiche des Stents umströmt werden. Dies kann zur Folge haben, dass eine Degradation des Trägers oder die Diffusion der Substanz in den Endbereichen erhöht ist. Bei jeder konventionellen medikamentösen Therapie werden optimale Dosierungen am Wirkungsort zur Unterstützung des Heilungsprozesses angestrebt. Dies muss aber auch auf lokaler Ebene gelten, wenn die Gewebsstrukturen in diesem lokalen Bereich eine divergierende Behandlung erfordern. So kann eine zu geringe Dosierung den Heilungsprozess nicht unterstützen und eine zu hohe Dosierung gar kontraproduktiv Ausgangspunkt für entzündliche Prozesse sein.

Als biodegradierbarer Träger können alle polymeren Matrizes synthetischer Natur oder natürlichem Ursprungs im erfindungsgemäßen Sinne eingesetzt werden, die aufgrund enzymatischer oder hydrolytischer Prozesse im lebenden Organismus abgebaut werden. Insbesondere können dazu Polymere aus der Gruppe Cellulose, Kollagen, Albumin, Casein, Polysaccharide (PSAC), Polylactid (PLA), Poly-L-lactid (PLLA), Polyglykol (PGA), Poly-D,L-lactid-co-glycolid (PDLLA/PGA), Polyhydroxybuttersäure (PHB), Polyhydroxyvaleriansäure (PHV), Polyalkylcarbonate, Polyorthoester, Polyethylenterephthalat (PET), Polymalonsäure (PML), Polyanhydride, Polyphosphazene, Polyaminosäuren und deren Copolymere sowie Hyaluronsäure eingesetzt werden. Die Polymere können je nach den gewünschten Eigenschaften des Beschichtungssystems in Reinform, in derivatisierter Form, in Form von Blends oder als Copolymere aufgebracht werden.

Als pharmakologisch wirksame Substanzen, die insbesondere zur Behandlung der Folgen perkutaner koronarer Interventionen dienen, eignen sich beispielsweise Calzium-Antagonisten, ACE-Hemmer, Antikoagulantien, Antiaggregantien, Fischöle, antiproliferative Substanzen, Immunsuppresiva, Cheomtherapeutika, antiinflammatorische Substanzen, Serotonin-Antagonisten sowie PPAR- und RXR-Agonisten.

Die Fig. 2a zeigt in einer stark schematisierten und vereinfachten Schnittansicht die Umlaufswandung 16, mit ihrem auf der äußeren Mantelfläche 18 aufgebrachten Beschichtungssystem 26. Das Beschichtungssystem 26 besteht aus zwei Endabschnitten 28.1 und 28.2 sowie einem mittleren Abschnitt 30. Im vorliegenden Falle wird das gesamte Beschichtungssystem 26 von einem in gleichmäßiger Schichtdicke aufgetragenen biodegradierbaren Träger und einer pharmakologisch wirksamen Substanz gebildet.

Die Abschnitte 28.1, 28.2, 30 unterscheiden sich dadurch, dass die pharmakologisch wirksame Substanz in höherer bzw. niedrigerer Konzentration in den Träger eingebracht ist. So ist im vorliegenden Fall die Konzentration der Substanz in den Endabschnitten 28.1, 28.2 gegenüber dem mittleren Abschnitt 30 erhöht. Wahlweise kann der Übergang von einer niedrigen Konzentration hin zu einer höheren Konzentration auch kontinuierlich über die gesamte Länge des Stents verlaufen.

Das in Fig. 1 dargestellte Beschichtungssystem 26 bietet sich insbesondere für zwei Fallkonstellationen an. Zum einen kann bei rheologischen Verhältnissen, die einen vermehrten Austrag der Substanz in den Endbereichen des Stents bedingen, eine weitestgehend gleichmäßige Dosierung über die gesamte Stentlänge gewährleistet werden. Zum anderen ist es möglich eine erhöhte Dosierung in den Endbereichen des Stents zu applizieren, so dass auf die pathophysiologischen Unterschiede des Gewebes über die gesamte Länge des Stents genauer eingegangen werden soll. So können in den Endbereichen insbesondere die Neointimabildung hemmende Substanzen in erhöhter Konzentration bereitgestellt werden.

Die Fig. 2b offenbart eine zweite Variante eines Beschichtungssystems 26 aus einem Träger und einer pharmakologisch wirksamen Substanz. Die Abschnitte 28.1, 28.2 entsprechen denen der Fig. 2a. Der Abschnitt 30 ist dagegen in seiner Schichtdicke deutlich reduziert. Dies hat zur Folge, dass die Dosierung der pharmakologisch wirksamen Substanz in den gegenüberliegenden Gewebsbereichen gemindert wird, d. h. insbesondere eine Dosierungsdauer verkürzt ist. Eine solche Schichtanordnung ist z. B. dann sinnvoll, wenn die pharmakologisch wirksame Substanz nur über einen kurzen Zeitraum den Bereich der Läsion erreichen soll und danach unter Umständen ein unerwünschter Einfluss auf den Heilungsprozess besteht.

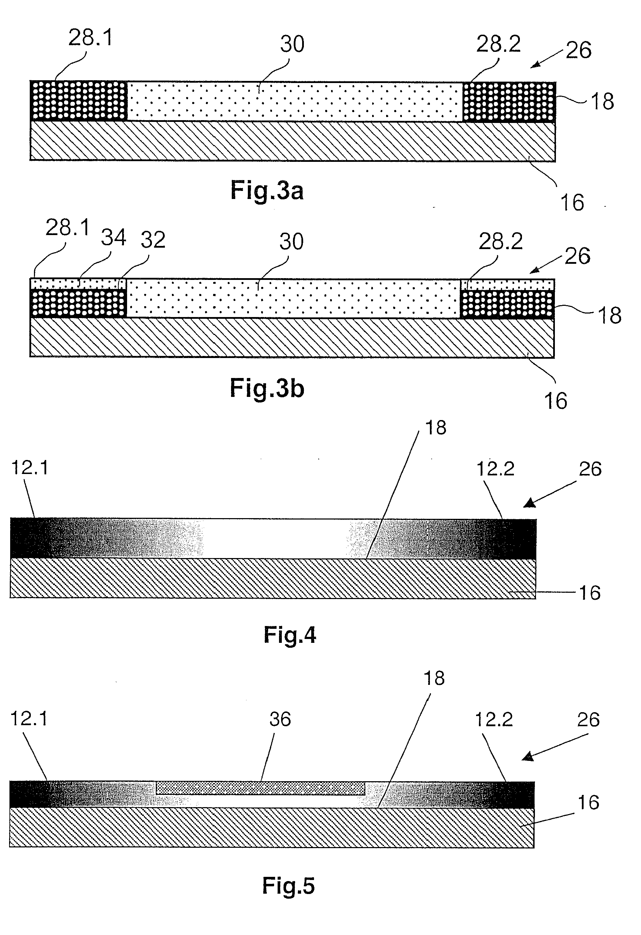

Der Fig. 3a ist ein Beschichtungssystem 26 zu entnehmen, bei der zwei verschiedene Träger, mit einem unterschiedlichen Degradationsverhalten in den Abschnitten 28.1, 28.2, 30 des Stents 10 aufgebracht sind. Ebenso verhält es sich bei der Variation des Systems nach Fig. 3b. In beiden Beschichtungssystemen 26 ist jeweils nur eine Substanz in über beide Träger homogener Konzentration verteilt.

Gemäß der Ausführung nach Fig. 3a werden die Abschnitte 28.1, 28.2 von einem Träger mit einem verzögerten Degradationsverhalten gegenüber dem Träger, der im mittleren Abschnitt 30 Einsatz findet, bedeckt. Dementsprechend wird die lokale Elutionscharakteristik der Substanz beeinflusst, d. h. in der Regel endseitig verzögert. Eine solche Ausführung ist immer dann sinnvoll, wenn die Dosierung an den Enden über einen längeren Zeitraum aufrecht erhalten werden soll, oder aufgrund der rheologischen Verhältnisse eine Austragung der Substanz entgegengewirkt werden soll.

Fig. 3b zeigt in den Abschnitten 28.1 und 28.2 ein in radialer Richtung mehrschichtigen Aufbau des Beschichtungssystems 26. In einem ersten Teilabschnitt 32 ist wiederum der Träger mit dem verzögerten Degradationsverhalten aufgetragen, während sich radial nach außen ein Teilabschnitt 34 mit dem schneller degradierbaren Träger befindet.

Der Fig. 4 ist ein Beschichtungssystem 26 zu entnehmen, bei dem auf einen einzigen Träger zwei unterschiedliche pharmakologisch wirksame Substanzen aufgebracht sind. Eine Konzentration der Substanzen ändert sich in axialer Richtung über die Länge des Stents kontinuierlich. Um den Konzentrationsverlauf der beiden Substanzen zu verdeutlichen wurde eine schematische Verlaufsdarstellung gewählt. Eine Konzentration einer ersten Substanz wird dabei über den Dunkelheitsverlauf, die einer zweiten Substanz über den Helligkeitsverlauf angedeutet. So ist eine Konzentration der ersten Substanz an den Enden 12.1, 12.2 des Stents stark erhöht, während seine Konzentration im mittleren Bereich stark abnimmt. Umgekehrt liegt die zweite Substanz in mittleren Bereichen des Beschichtungssystems 26 in erhöhter Konzentration vor und nimmt in Richtung der Enden 12.1 und 12.2 ab. Ein solches System eignet sich beispielsweise dazu, mit Hilfe der ersten Substanz eine lokal differenzierte medikamentöse Behandlung an den Enden 12.1 und 12.2 des Stents durchzuführen und im mittleren Bereich des Stents mit der zweiten Substanz im Wesentlichen die Läsion zu behandeln.

In der Fig. 5 ist das Beschichtungssystem 26 der Fig. 4 noch weiter differenziert worden, indem ein zusätzlicher mittlerer Teilabschnitt 36 aus einem anderen Träger, der zu dem eine weitere Substanz enthält, in das Beschichtungssystem 26 integriert ist. Der Träger des Teilabschnittes 36 zeigt ein sehr rasches Degradationsverhalten und setzt demnach die in ihm eingebettete weitere Substanz sehr rasch und mit hoher Dosierung frei. Anschließend werden die ersten und zweiten Substanzen wie bereits unter Fig. 4 geschildert, freigesetzt.

Die vorgenannten Beispiele der Fig. 2a, 2b, 3a, 3b, 4 und 5 stellen nur stark schematisierte Ausführungsbeispiele des erfindungsgemäßen Beschichtungssystems 26 dar. Sie können in vielfältiger Weise untereinander kombiniert werden. So ist es beispielsweise denkbar ein komplexes Beschichtungssystem zu entwerfen, das in einzelnen Abschnitten aus jeweils mehreren Trägersystemen mit jeweils verschiedenen Substanzen besteht. Primäres Ziel ist es dabei immer die lokale Dosierung der Substanzen in den gegenüberliegenden Gewebsabschnitten zu optimieren.

## Patentansprüche

1. Stent (10) mit einem rohrförmigen, an seinen Stirnseiten offenen Grundkörper (14), dessen Umlaufswandung (16) zumindest bereichsweise mit einem Beschichtungssystem (26) aus einem oder mehreren polymeren Trägern und wenigstens zwei pharmakologisch wirksamen Substanzen bedeckt ist, wobei die Substanzen nach Implantation des Stents (10) in den menschlichen oder tierischen Körper in das umgebende Gewebe freigesetzt werden,
**dadurch gekennzeichnet, dass**
die Konzentration der wenigstens zwei pharmakologisch wirksamen Substanzen in Längsrichtung des Stents (10) derart vorgegeben ist, dass die Substanz eine in Abhängigkeit von den in der Applikation zu erwartenden pathophysiologischen und/oder rheologischen Verhältnissen vorgegebene, in Längsrichtung des Stents (10) lokal unterschiedliche Elutionscharakteristik aufweist.

2. Stent (10) nach Anspruch 1, **dadurch gekennzeichnet, dass** der oder die polymeren Träger biodegradierbar sind.

3. Stent (10) nach Anspruch 2, **dadurch gekennzeichnet, dass** ein Degradationsverhalten des oder der Träger zur Differenzierung der lokalen Elutionscharakteristik der Substanzen dient.

4. Stent (10) nach einem der Ansprüche 1 bis 3, **dadurch gekennzeichnet, dass** auf einem polymeren Träger des Beschichtungssystems (26) eine erste und eine zweite pharmakologisch wirksame Substanz aufgebracht sind, wobei die Konzentration der beiden Substanzen in Längsrichtung des Stents unterschiedlich ist, und sich in Längsrichtung des Stents kontinuierlich ändert.

5. Stent (10) nach Anspruch 4, **dadurch gekennzeichnet, dass** auf dem polymeren Träger des Beschichtungssystems (26) der Konzentrationsverlauf der ersten pharmakologisch wirksamen Substanz in Längsrichtung des Stents entgegengesetzt zum Konzentrationsverlauf der zweiten pharmakologisch wirksamen Substanz verläuft, so dass ein kontinuierlicher Übergang von Abschnitten erhöhter Konzentration der ersten pharmakologisch wirksamen Substanz zu Abschnitten erhöhter Konzentration der zweiten pharmakologisch wirksamen Substanz besteht.

6. Stent (10) nach Anspruch 5, **dadurch gekennzeichnet, dass** sich auf dem polymeren Träger des Beschichtungssystems (26) die Abschnitte erhöhter Konzentration der ersten pharmakologisch wirksamen Substanz an den Stentenden (12.1, 12.2) befinden und deren Konzentration im mittleren Bereich des Stents stark abnimmt, während sich der Abschnitt erhöhter Konzentration der zweiten pharmakologisch wirksamen Substanz im mittleren Bereich des Stents befindet und sich deren Konzentration zu den Stentenden hin stark abnimmt.

7. Stent (10) nach einem der Ansprüche 4 bis 6, **dadurch gekennzeichnet, dass** in das Beschichtungssystem (26) ein zusätzlicher Teilabschnitt (36) bestehend aus einem Träger integriert ist, welcher eine dritte pharmakologisch wirksame Substanz enthält, wobei sich der zusätzliche Teilabschnitt vorzugsweise im mittleren Bereich des Stents befindet.

8. Stent (10) nach Anspruch 7, **dadurch gekennzeichnet, dass** der Träger des Teilabschnittes (36) ein rasches Degradationsverhalten aufweist und die dritte Substanz rasch und in hoher Dosierung freisetzt wird.
